(19) Europäisches Patentamt
European Patent Office
Office européen des brevets

(11) Publication number: 0 122 028 A1

# EUROPEAN PATENT APPLICATION

(12)

(21) Application number: 84301525.6

(22) Date of filing: 07.03.84

(51) Int. Cl.³: C 12 Q 1/00, C 12 Q 1/04, C 12 Q 1/36

(30) Priority: 07.03.83 US 472664

(43) Date of publication of application: 17.10.84 Bulletin 84/42

(84) Designated Contracting States: AT BE CH DE FR GB IT LI LU NL SE

(71) Applicant: E-Y LABORATORIES, INC., 127 North Amphlett Boulevard, San Mateo California 94401 (US)

(72) Inventor: Chun, Peter Kwok Chi, 2566 Adams Court, South San Francisco California 94080 (US)
Inventor: Chu, Albert En-Yuen, 819 Maple Street, San Mateo California 94402 (US)

(74) Representative: Bizley, Richard Edward et al, BOULT, WADE & TENNANT 27 Furnival Street, London EC4A 1PQ (GB)

(54) **Colorimetric assay for enzymes, diagnostic article therefor and a method for forming such article.**

(57) A method for the colorimetric assay of a suspected enzyme-containing substance in a biologically derived specimen is provided by absorbing a substrate for the enzyme onto an absorbent surface such as a swab, which is contacted with the specimen to produce a color if the enzyme is present. Multiple substrates producing different colors may be absorbed onto the swab for the detection of multiple enzymes. The invention includes a diagnostic article, and its formation, incorporating this principle.

EP 0 122 028 A1

# COLORIMETRIC ASSAY FOR ENZYMES, DIAGNOSTIC ARTICLE THEREFOR AND A METHOD FOR FORMING SUCH ARTICLE

The present invention relates to the colorimetric detection of enzyme activity in biologically derived specimens, and particularly to the detection of such activity in microorganisms such as bacteria, using a substrate specific for the enzyme.

Simple and rapid assays for bacteria and other microorganisms or substances in biologically derived materials (e.g. serum, urine, surface fluids, exudates, or from culture media or culture broth) are important tools in diagnosis. It is particularly advantageous to provide such assays wherein a doctor or trained technician can make a diagnosis without independent laboratory work. It is well known that certain substrates are specifically reactive with enzymes of bacteria and the like, to change from a colorless to a colored form, or from one color to another, upon incubation with such enzymes. For example, a test is described for urease as a presumptive identification of yeast-like organisms. [Zimmer, B. L., et al, J. Clin. Microbiol., Vol. 10, p. 380 (1979).] That publication describes the absorption of urea and an indicator onto a cotton-tipped applicator, which is then dried. Thereafter, the applicator is inoculated with colonies of the test organism suspected of containing urease. The inoculated applicator is placed into a test tube and changes color from yellow to purple when the pH of the solution is lowered due to the hydrolysis of urea in the presence of urease.

In accordance with the present invention, a method is provided for the colorimetric assay of one or more enzymes in enzyme-containing substances in a biologically derived specimen. A diagnostic article for an assay of a single

enzyme is first formed by absorbing a substrate for certain enzymes onto an absorbent surface. Particularly effective substrates are for oxidases, glycosidases, esterases, phosphatases, arylsulfatases, beta-lactamases, DNA'ases, iminopeptidases, and aminopeptidases. A preferred surface is the surface of an absorbent swab. The swab surface is dried and is ready for use in the colorimetric assay.

During the assay of a single enzyme, the specimen, which possibly includes suspected enzyme-containing substance, is absorbed onto the absorbent surface. Then the absorbent surface is incubated, preferably in a moist atmosphere. The presence or alteration of a color on the surface after incubation is a positive indication that the specimen includes the suspected substance. In some instances, additional reagent such as a diazo dye is required for full color development. For the aminopeptidase enzyme, a particularly fast and thus effective substrate is a gamma glutamyl naphthylamide, substituted at the 4-position, the 6-position, or both, with an electron donating group.

The system is applicable to the simultaneous detection of multiple enzymes in a single biologically derived sample using a single absorbent surface. Thus, at least a first and second substrate are absorbed onto the absorbent surface. The first substrate is capable of producing a first color upon incubation in the presence of a first enzyme, while the second substrate is capable of producing a second color upon incubation in the presence of a second enzyme. The first color is selected to be distinct from the second color, and the combination of the first and second colors form a third color distinct from either the first or second colors. Thus, upon incubation, a change to the first, second, or third colors are positive indications that the specimen includes the first enzyme, the second enzyme, or both enzymes, respectively. The color formation may be accomplished by conversion of the substrate itself to the color, by conversion of the substrate with a diazo dye developer after being converted in the presence of the enzyme to an appropriate form.

Thus, the present invention relates to the colorimetric assay of one or more suspected enzyme-containing substances in a biologically derived specimen such as urine, serum, plasma, exudates, or from a fluid on a surface of the body such as the ear, throat, or external skin, genitalia, or from a culture medium or broth.

Briefly summarized, the diagnostic article used for the colorimetric assay of the present invention is formed as follows. One or more substrates for each enzyme to be detected in a suspected enzyme-containing substance are absorbed onto an absorbent surface. A particularly effective and convenient absorbent surface comprises the surface of a porous swab attached to a handle, such as a stick. The swab may be formed of any suitable absorbent material such as cotton, polyester, fiberglass filament, or the like. Typically, the substrate is dissolved in an aqueous or organic solution prior to absorption. Suitably, 0.05 to 1.0 ml of solution containing 1 mg/ml of substrate in aqueous, buffered, or alcohol solution can be used for each swab. The absorbent surface is preferably dried, as in air, prior to use in the colorimetric assay.

In a typical instance, the use of the substrate-containing absorbent surface in the colorimetric assay is as follows. The swab is contacted with the specimen suspected of containing certain bacteria. Preferably, the specimen is first incubated or cultured in a suitable media or broth to increase the concentration of bacteria prior to contact with the absorbent surface.

After incubation of the bacteria, the absorbent surface is contacted with the bacteria and is incubated under appropriate conditions such as a moist atmosphere, typically in an isolated area. If the color of the absorbent surface is altered from colorless to colored, or from one color to another in the specified manner, this is a positive indication that the specimen contains the bacteria with the enzyme to which the substrate is specific.

Any substrate may be used which is capable of absorption onto the absorbent surface, and which is also capable of producing a specific color only upon incubation in the presence of the enzyme portion of the suspected enzyme-containing substance. In some instances, the substrate itself is converted either from one color to another, or from a colorless form to a colored form, in the presence of the enzyme. In other instances, the substrate is converted by the enzyme to a form which, in turn, is coupled with a diazo dye prior to colorimetric detection.

Suitable enzyme-substrate systems are set forth in the following Table.

TABLE

| ENZYME | SUBSTRATE |
| --- | --- |
| 1. GLYCOSIDASES: (A family of enzymes which hydrolyze glycosidic bonds) (e.g. alpha-mannosidase, beta-galactosidase, alpha-glucosidase) | (a) Color-producing moiety<br>alpha and beta 6-bromo-naphthyl,<br>alpha and beta naphthyl,<br>alpha and beta 4-methoxy naphthyl,<br>p-nitrophenol,<br>o-nitrophenol,<br>5-Bromo-4-chloro-3-indolyl,<br>bromothymolphthalein,<br>phenolphthalein,<br>4-methylumbelliferyl, or<br>fluorescein<br>(b) Sugar derivative moiety<br>Sugars: dextro and levo-rotatary, alpha and beta anomers of:<br>(i) glycosides (e.g. alpha-D-glucopyranoside),<br>(ii) aminoglycosides(e.g.D-galactosamine),<br>(iii) amino-acyl-glycosides (e.g. N-acetyl-D-glucosamine),<br>(iv) sugar acids (aldonic, aldaric and uronic acids) (e.g. D-gluconic acid D-glucaric acid D-glucoronic acid), or<br>(v) sialic acids (e.g. N-Acetyl-neuraminic acid) |
| 2. ESTERASES: (A family of enzymes which hydrolyze ester bonds) | (a) Color-producing moiety<br>alpha and beta 6-bromo-naphthyl,<br>alpha and beta naphthyl,<br>alpha and beta 4-methoxy naphthyl,<br>p-nitrophenol,<br>o-nitrophenol,<br>5-bromo-4-chloro-3-indolyl, |

| | |
|---|---|
| 2. ESTERASES: | (a) Color-producing moiety (cont'd) bromothymolphthalein, phenolphthalein, 4-methylumbelliferyl, or fluorescein |
| | (b) Carboxylate moiety Saturated and/or unsaturated, branched and/or straight chain carboxylic acids of varying carbon numbers, and their salts: (e.g. butyrate, acetate, caprate, caproate, caprylate, myristate, laurate, palmitate, oleate, valerate etc.) |
| 3. PHOSPHATASES: (e.g. acid phosphatase and alkaline phosphatase) | Color-producing moieties of phosphate alpha and beta 6-bromo-naphthyl, alpha and beta naphthyl, alpha and beta 4-methoxy naphthyl, p-nitrophenol, o-nitrophenol, 5-Bromo-4-chloro-3-indolyl, bromothymolphthalein, phenolphthalein, 4-methylumbelliferyl, or fluorescein |
| 4. ARYLSULFATASES: | Color-producing moieties of sulfate alpha and beta 6-bromo-naphthyl, alpha and beta naphthyl, alpha and beta 4-methoxy naphthyl, p-nitrophenol, o-nitrophenol, 5-Bromo-4-chloro-3-indolyl, bromothymolphthalein, phenolphthalein, 4-methylumbelliferyl, or fluorescein |

| | |
|---|---|
| 5. AMINOPEPTIDASES and IMINOPEPTIDASES: (A family of enzymes which hydrolyze amide bonds and whose specificity is governed by the amino acid to which the amide bond is adjacent to - e.g., gamma-glutamylaminopeptidase) | (a) Color-producing moiety<br>alpha and beta alkoxyl-naphthyl derivatives, and<br>alpha and beta naphthyl or 7-amido-4-methyl coumarin<br>(b) Peptide moiety<br>Amino acids and dipeptides and tripeptides |
| 6. BETA-LACTAMASES: | (7-(thienyl-2-acetamido)-3(2(4-N,N-dimethylaminophenylazo)pyridinium methyl)-3-cephem-4-carboxylic acid) |
| 7. OXIDASES: | N,N-dimethyl-p-phenylenediamine monohydrochloride |
| 8. DNA'ases: | 5-bromo-4-chloro-3-indoyl-thymidine-3-phosphate |

In the above Table, certain enzyme-substrate systems require coupling with a diazo dye. They include the naphthol substrates, the iminopeptidases, and aminopeptidases and their enzymes. Amino acid naphthylamides are known substrates for aminopeptidases. For example, it is well known that gamma glutamyl-naphthylamide is a specific substrate for gamma glutamyl aminopeptidase. Thus, in general, the specific amino acid naphthylamides form substrates for the corresponding aminopeptidases. Conventional substrates of the above type require substantial times of incubation periods for colorimetric detection through coupling with a diazo dye. For the present absorbent surface technique, it is preferable to speed up this coupling reaction. Briefly, the speed is vastly increased by the substitution of an electron donating group R (e.g. an alkoxy group, such as methoxy, an hydroxy group, or a halogen) at the 4-position, the 6-position, or both, by reference to the following structure.

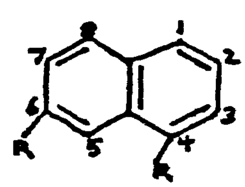

Specific enzyme-substrate systems of this type, and suitable diazo coupling dyes for converting substrate-enzyme reaction products to colored form, are set forth below. Reference is also made to our co-pending European patent application filed today (a copy of which is filed herewith) entitled "Microorganism Detection Methods and the Separation of Agglutinated Microorganisms, and a Kit for use therein".

Thus, an effective susbtrate for the gamma-glutamyl aminopeptidase enzyme of Neisseria meningitidis is gamma-glutamyl-4-alkoxy-beta-naphthylamide, specifically with methoxy as the alkoxy group. Using this substrate and a suitable diazo coupler, the color changes in less than 10 minutes at an incubation temperature of 37°C for the same concentrations of reactants.

An effective substrate for the proline aminopeptidase enzyme portion of Neissieria gonorrhoeae is a fast substrate comprising proline-beta-naphthylamide substituted at the 4-position, 6-position, or both, with an electron donating group of the foregoing type. A particularly effective substrate comprises proline-4-alkoxy-beta-naphthylamide, specifically the 4-methoxy method.

A fast substrate for the glycyl-prolyl-aminopeptidase enzyme portion of Neisseria gonorrhoeae comprises glycyl-prolyl-naphthylamide substituted at the 4-position, the 6-position, or both, with an electron donating group of the foregoing type. A particularly effective substrate is glycyl-prolyl-4-alkoxy-beta-naphthylamide, specifically the 4-methoxy compound.

Suitable diazo coupling dyes for converting substrate-enzyme reaction products to colored form include the following: 4-amino-2,5-dimethoxy-4'-nitroazo-benzene diazonium salt; tetrazotized o-dianisidine; diazotized-4'-amino-2',5'-diethoxybenzanilide zinc chloride salt; diazotized product of 4-benzoylamino-2,5-dimethoxyaniline zinc chloride; o-aminoazotoluene, diazonium salt (known as Fast Garnet); anthraquinone-1-diazonium chloride; 5-nitro-2-amino-methoxybenzene diazotate; N', N'-diethyl-4-methoxymethanilamide, diazonium salt; 2-amino-4-methoxybenzamide, diazonium salt; diazo-2-amino-5-chloroanisole, or diazo-5-chloro-o-anisidine; 5-chloro-2-toluidinediazonium chloride hemi-

zinc chloride; 5-chloro-4-benzamido-2-methylbenzene diazonium chloride, hemi-zinc chloride; 6-benzamido-5-methoxy-m-toluidine diazonium chloride; and other diazonium salts.

The present invention is applicable to the detection of multiple enzymes in a single specimen, utilizing a single absorbent surface such as a swab. One such system includes a test for a first and second enzyme contained in a biologically derived specimen. Here, at least a first and second substrate are first absorbed onto the absorbent surface. The first substrate is capable of producing a first color upon incubation in the presence of the first enzyme, while the second substrate is capable of producing a second color upon incubation in the presence of the second enzyme. The first color is selected to be distinct from the second color, and the combination of the first and second colors form a third color, which is also distinct from either the first or second colors. In the test, the absorbent surface is contacted with the specimen and then incubated. One of four possibilities exist -- no color conversion, conversion to the first color, conversion to the second color, or conversion to the third color; each possibility being a presumptive indication of a different enzyme activity condition. If there is no color conversion, then presumptively neither the first nor second enzyme is present. If the first color is present after incubation, then presumptively the only enzyme activity is caused by the first enzyme. If the second color is present, then presumptively only the second enzyme activity is present, while if the third color is present after incubation, then presumptively both enzymes are present. By way of a specific example, both substrates may be colorless in the absence of either enzyme, colored yellow in the presence of the first enzyme but not the second enzyme, and colored blue in the presence of the second enzyme but not the first enzyme. In this instance, a colorless final product would indicate no enzyme activity present, a yellow color would indicate the presence of the first enzyme activity, a blue color the presence of the second enzyme activity, and a green color enzyme activity for both the first and second enzymes.

A number of variations of the above multiple enzyme aspect of the invention are possible. For example, one of the substrates may be directly convertible to colored form in the presence of the enzyme, while a second substrate is colorless unless activated as by coupling with a diazo dye. The combinations of the direct conversion substrates and the diazo coupling substrates provide a rapid scheme for detecting three or more enzymes using three or more substrates on a single absorbent surface. Thus, for example, the first substrate may be of a direct

conversion type from colorless to yellow, such as O-nitrophenyl beta galacto-pyranoside; the second substrate being of a direct conversion type from colorless to blue, such as 5-bromo-4-chloro-3-indoyl beta glucuronide; and the third substrate requiring diazo dye coupling, e.g. an amino acid naphthylamide, which converts from colorless to red form in the presence of a diazo dye. In this instance, the initial incubation is performed in the absence of the diazo dye coupler. If the surface is colorless, this indicates the absence of the enzyme corresponding to the first or second substrates. Then the diazo dye is added, and a red color is a positive indication of the presence of the third enzyme. If a yellow color develops prior to the addition of the diazo dye, this is an indication of the enzyme which corresponds to the first substrate. Upon development with the diazo dye, an orange color is indicative of the presence of the enzyme for the third substrate as well. Similarly, an initial blue color is indicative of the presence of the second enzyme, and a purple color of the presence of the third enzyme as well. Finally, a green initial color is indicative of the presence of both the first and second enzymes, while after development with the diazo dye, a combined color of green and red is indicative of the presence of the third enzyme as well.

It is apparent from the foregoing that the present system is applicable to a variety of multiple substrate-multiple enzyme tests on a solid absorbent surface using a variation of the above types of substrate-enzyme systems. The ability to test for multiple enzyme activities on a single absorbent surface, such as a swab, provides a number of unique advantages. For example, frequently there is a limited amount of specimen available for testing, and the ability to perform multiple tests at minimum specimen is a distinct advantage. In addition, reagents are conserved, and testing time is minimized.

Specific examples illustrating the present invention are as follows.

Example 1

PADAC (7-(thienyl-2-acetamido)-3(2(4-N,N-dimethylaminophenylazo)pyridinium methyl)-3-cephem-4-carboxylic acid) is a purple substrate that changes to yellow color when acted upon by beta-lactamase. A cotton swab is impregnated with a 0.1 ml of an aqueous solution containing 0.1 mg/ml of this substrate. The swab is then air dried.

The swab is used to pick up a bacterial colony suspected to be Neisseria gonorrhoea, from a culture plate. The swab is then wet with 2 drops of water

and allowed to incubate in a 37°C incubator for 10 to 15 minutes. A color change from purple to yellow indicates the presence of beta-lactamase, and hence that the Neisseria gonorrhoea is a beta-lactamase producing strain. If no color change occurs, then the Neisseria gonorrhoea is a non beta-lactamase producing strain.

Example 2

A cotton swab is impregnated with 0.1 ml of a 0.05 M phosphate buffer containing 1 mg/ml of o-nitrophenyl-D-galactopyranoside and 1 mg/ml of gamma-glutamyl 4-methoxy-beta-naphthylamide. The swab is then air dried.

The swab is used to pick up a few morphologically similar cultures which have been grown on Thayer Martin culture medium and have been tested and found to be gram negative diplococci, oxidase positive organisms.

The swab is moistened by 2 drops of water and incubated in a 37°C incubator for 10 minutes. The color of the swab, which is originally colorless, is checked for any color change. A yellow color indicates the presence of beta-galactosidase and hence Neisseria lactamica; no color change indicates the possibility of Neisseria gonorrhoea, Neisseria meningitidis, or Branhamella catarrhalis. At this point, when no color change occurs, a drop of Fast Garnet GBC salt (diazo dye) solution (1 mg/ml aqueous solution) is added to the swab. A color change to red indicates the presence of gamma-glutamyl aminopeptidase, and hence Neisseria meningitidis. No color change after addition of the Fast Garnet GBC indicates the possible presence of Neisseria gonorrhoea or Branhamella catarrhalis.

Example 3

Background

The family Enterobacteriaceae comprises the species from Escherichia, Shigella, Salmonella, Arizona, Citrobacter, Klebsiella, Enterobacter, Serratia, Proteus and Providencia. The group can be divided largely by biochemical reactions by the presence of three enzyme activities:

(1) Citrobacter, Klebsiella, Enterobacter, some Serratia, Escherichia and Arizona have beta-galactosidase. A substrate for beta-galactosidase

is o-nitrophenol-beta-D-galactopyranoside, which changes from colorless to yellow when acted upon by this enzyme.

(2) Escherichia and some Shigella have beta-glucuronidase. A substrate for beta-glucuronidase is 5-bromo-4-chloro-3-indoyl-beta-glucuronide, which changes to a blue color when acted upon by this enzyme.

(3) Citrobacter, Klebsiella, Enterobacter and Serratia have pyrrolidonyl peptidase activity. A substrate for the enzyme is L-pyrrolidonyl-beta-4-methoxy naphthylamide, which when acted upon by this enzyme releases a product that changes red when Fast Garnet GBC is added.

Procedure

A cotton swab is impregnated with 100 µl of a solution containing 1 mg/ml each of o-nitrophenyl-beta-D-galactopyranoside, 5-bromo-4-chloro-3-indoyl-beta-glucoronide, and L-pyrrolidonyl-beta-4-methoxy naphthylamide, and then air dried.

The swab is used to pick up a few colonies suspected to belong to the Enterobacteriaceae family. The swab is then moistened by 2 drops of water and allowed to incubate in a 37°C incubator for 60 minutes. Any color changes are observed, and then 1 drop of Fast Garnet GBC (a diazo coupler) salt solution (1 mg/ml aqueous solution) is added to the swab, and any color change after this is again noted. There are several possibilities that can occur:

| Colors | Interpretation |
| --- | --- |
| 1. Yellow color, and then red/orange after adding Fast Garnet | Beta-galactosidase and pyrrolidonyl peptidase present (suspect Citrobacter, Klebsiella, Enterobacter or Serratia) |
| 2. Blue color, then red/purple after adding Fast Garnet | Beta-glucuronidase and pyrrolidonyl peptidase present (probably not Enterobacteriaceae) |
| 3. Green color, then red/brown after adding Fast Garnet (green is a combination of yellow and blue colors) | Beta-glucuronide and beta-galactosidase and pyrrolidonyl peptidase present (probably not Enterobacteriaceae) |
| 4. No color change, then red after adding Fast Garnet | Only pyrrolidonyl peptidase present (suspect Serratia) |

| Colors | Interpretation |
|---|---|
| 5. Yellow color, followed by no red color after adding Fast Garnet | Beta-galactosidase present only (suspect Arizona) |
| 6. Blue color, followed by no red color after adding Fast Garnet | Beta-glucuronidase present only (suspect Shigella) |
| 7. Green color, followed by no red color after adding Fast Garnet | Beta-glactosidase and beta-flucuronidase present only (suspect E. coli) |
| 8. No color change, and no color change after adding Fast Garnet | None of the three enzymes present (suspect Salmonella, Shigella, Proteus or Providencia) |

This test is a very useful test because in many situations clinical microbiologists are interested only in knowing whether the culture contains Salmonella and Shigella. This method allows for rapid screening.

CLAIMS:

1. A method for the colorimetric assay of a suspected enzyme-containing substance in a a biologically derived specimen, comprising absorbing the specimen, possibly including said suspected enzyme-containing substance, onto an absorbent surface containing an absorbed substrate for the enzyme of said suspected enzyme-containing substance, said substrate being capable of producing a specific color only upon incubation in the presence of said enzyme, said enzyme being an oxidase, glycosidase, esterase, phosphatase, arylsulfatase, beta-lactamase, DNA'ase, iminopeptidase, or aminopeptidase, and then incubating said absorbent surface.

2. A method as claimed in claim 1 in which said suspected enzyme-containing substance is bacteria.

3. A method as claimed in claim 1 or claim 2 in which said specific color is formed by conversion of said substrate itself to said specific color or by coupling of said substrate with a diazo dye developer.

4. A method as claimed in any one of claims 1 to 3 in which said absorbent surface comprises the surface of a swab.

5. A method as claimed in any one of claims 1 to 4 in which after absorbing the specimen onto the absorbent surface, said absorbent surface is removed to a separate zone for incubation.

6. A method as claimed in claim 1 in which the enzyme comprises an aminopeptidase or iminopeptidase, and said substrate is a fast substrate comprising amino acid naphthylamide substituted at the 4-position, the 6-position, or both, with an electron donating group.

7. A method for the colorimetric assay of at least a first and second enzyme contained in a biologically derived specimen, comprising absorbing the specimen, possibly containing said first and second enzymes, onto an

absorbent surface containing at least first and second absorbed substrates, said first substrate being capable of producing a first color upon incubation in the presence of said first enzyme, said second substrate being capable of producing a seconc color upon incubation in the presence of said second enzyme, said first color being distinct from said second color, and the combination of said first and second colors forming a third color distinct from said first and second colors, and then incubating said absorbent surface.

8. A method as claimed in claim 7 in which said first color or said second color is formed by conversion of said first substrate itself to said first color or by conversion of said second substrate itself to said second color, respectively.

9. A method as claimed in claim 8 in which said second color is formed by conversion of said substrate with a diazo dye developer, said first color being formed by conversion of said first substrate itself to said first color.

10. A method as claimed in claim 9 in which said diazo dye developer is contacted with said absorbent surface subsequent to incubation for development of said first color.

11. A method for forming a diagnostic article for a colorimetric assay comprising:

(a) absorbing onto an absorbent surface, e.g. the surface of a swab, a substrate for the enzyme of a suspected enzyme-containing substance in a biologically derived specimen, said substrate being contained in a solution and being capable of producing a specific color only upon incubation in the presence of said enzyme, said enzyme being an oxidase, esterase, phosphatase, arylsulfatase, beta-lactamase, DNA'ase, iminopeptidase or aminopeptidase, and

(b) drying said absorbent surface.

12. A method as claimed in claim 11 in which at least first and second different substrates are absorbed onto said absorbent surface, said first substrate being capable or producing a first color upon incubation in the presence of a first enzyme and said second substrate being capable of producing a second color distinct from said first color in the presence of a second enzyme.

13. A method as claimed in claim 11 or claim 12 in which the enzyme(s) comprise(s) an aminopeptidase or iminopeptidase, and said respective substrate is a fast substrate comprising amino acid naphthylamide, e.g. gamma-glutamyl naphthylamide, substituted at the 4-position, the 6-position, or both, with an electron donating group.

14. A diagnostic article for the colorimetric assay of a suspected enzyme-containing substance in a biologically derived specimen comprising an absorbent surface, e.g. the surface of a swab attached to a handle, upon which is absorbed a substrate specific for the enzyme of a suspected enzyme-containing substance, said substrate being capable of producing a specific color only upon incubation in the presence of said enzyme, said enzyme being an oxidase, glycosidase, esterase, phosphatase, arylsulfatase, beta-lactamase, DNA'ase, iminopeptidase or aminopeptidase.

15. An article as claimed in claim 14 and further defined by the specific feature of claim 12 and/or claim 13.

0122028

European Patent Office

# EUROPEAN SEARCH REPORT

Application number

EP 84 30 1525

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 018 825 (NATIONAL RESEARCH DEVELOPMENT CORP.) * Whole document * --- | 1-6,11,13-15 | C 12 Q 1/00<br>C 12 Q 1/04<br>C 12 Q 1/36 |
| Y | US-A-4 018 653 (F.C. MENNEN) * Whole document * --- | 1,4,5,11,14,15 | |
| Y | GB-A-2 031 949 (AMERICAN HOME PRODUCTS CORP.) * Pages 3-6; claims 1-15 * --- | 1-3,6-9,11-14 | |
| A | US-A-4 390 622 (G.W. CARTWRIGHT) * Whole document * --- | 1,2,11,14 | TECHNICAL FIELDS SEARCHED (Int. Cl. 3) |
| A | US-A-3 862 011 (R.E. SMITH) * Whole document * --- | 6,13 | C 12 Q |
| A | GB-A-2 050 418 (LABORATOIRE DE RECHERCHE API) * Whole document * --- | 1,2,11,14 | |
| A | GB-A-2 093 994 (NATIONAL RESEARCH DEVELOPMENT CORP.) ----- | | |

The present search report has been drawn up for all claims

| Place of search | Date of completion of the search | Examiner |
|---|---|---|
| THE HAGUE | 22-06-1984 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82